(19)

Europäisches
Patentamt
European
Patent Office
Office européen
des brevets

(11) **EP 2 365 960 B1**

(12) **EUROPEAN PATENT SPECIFICATION**

(45) Date of publication and mention
of the grant of the patent:
**20.11.2013 Bulletin 2013/47**

(21) Application number: **09760585.1**

(22) Date of filing: **29.10.2009**

(51) Int Cl.:
**C07C 201/12** [(2006.01)]     **C07C 205/11** [(2006.01)]

(86) International application number:
**PCT/IB2009/007264**

(87) International publication number:
**WO 2010/052536 (14.05.2010 Gazette 2010/19)**

(54) **Process for the preparation of para-nitrobenzyl bromide**

Verfahren zur Herstellung von para-Nitrobenzylbromid

Procédé pour la préparation de bromure de para-nitrobenzyle

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **06.11.2008 IN DE25132008**

(43) Date of publication of application:
**21.09.2011 Bulletin 2011/38**

(73) Proprietor: **Council of Scientific & Industrial
Research
New Delhi 110 001 (IN)**

(72) Inventors:
• **AGRAWAL, Manoj, Kunjabihari
Gujarat (IN)**
• **GHOSH, Pushpito, Kumar
Gujarat (IN)**
• **GANDHI, Maheshkumar, Ramniklal
Gujarat (IN)**
• **UPADHYAY, Sumesh, Chandra
Gujarat (IN)**
• **ADIMURTHY, Subbarayappa
Gujarat (IN)**
• **RAMACHANDRAIAH, Gadde
Gujarat (IN)**
• **PATOLIYA, Paresh, Ukabhai
Gujarat (IN)**
• **JOSHI, Girdhar**

  **(IN)**
• **BRAHMBHATT, Harshad
Gujarat (IN)**

• **SANGHAVI, Rahul, Jasvantrai
Gujarat (IN)**

(74) Representative: **Noel, Chantal Odile et al
Cabinet Orès
36, rue de St Pétersbourg
75008 Paris (FR)**

(56) References cited:
**JP-A- 2 221 233      JP-A- 11 080 035
US-A1- 2003 136 941**

• **ADIMURTHY SUBBARAYAPPA ET AL: "An
alternative method for the regio- and
stereoselective bromination of alkenes, alkynes,
toluene derivatives and ketones using a bromide/
bromate couple" GREEN CHEMISTRY, ROYAL
SOCIETY OF CHEMISTRY, CAMBRIDGE, GB, vol.
10, no. 2, 1 January 2008 (2008-01-01), pages
232-237, XP008119264 ISSN: 1463-9262**
• **H.M. BELL, H.C. BROWN: "Selective Reductions.
XI. The Reaction of Sodium Borohydride with
Alkyl Halides under Solvolytic Conditions.
Borohydride as a Convenient Trap for Carbonium
Ions" JOURNAL OF THE AMERICAN CHEMICAL
SOCIETY, vol. 88, 1966, pages 1473-1477,
XP002570106 DOI: 10.1021/ja00959a020**
• **MESTRES RAMON ET AL: "High atomic yield
bromine-less benzylic bromination" GREEN
CHEMISTRY, ROYAL SOCIETY OF CHEMISTRY,
CAMBRIDGE, GB, vol. 4, no. 4, 1 January 2002
(2002-01-01) , pages 314-316, XP008119279 ISSN:
1463-9262 [retrieved on 2002-06-27]**

**Description**

**FIELD OF THE INVENTION**

The present invention relates to an improved process for the preparation of *p*-nitrobenzyl bromide. The compound is widely used for esterification of carboxylic acids especially where deprotection is required such as in the synthesis of β-lactam antibiotic analogs, cephalosporine analogs, penicillin ester analogs, fusidic acid analogs and thienamycin analogs. It is also used as a starting material in the preparation of the drug rizatriptan benzoate.

**BACKGROUND OF THE INVENTION**

Reference may be made to the paper entitled "Bromination of p-nitrotoluene" by J. F. Brewster (J. Am. Chem. Soc.; 1918; 40(2); 406-407). The reaction was carried out by reacting *p*-nitrotoluene with liquid bromine in carbon tetrachloride at elevated temperature (on a hot plate) with exposure to sunlight. *p*-nitrobenzyl bromide was obtained in 71 % overall yield. The bromine atom efficiency was computed to be 40%.

Reference may be made to the procedure reported by G. H. Coleman et. al. in Organic Syntheses, Coll. Vol. 2, p.443 (1943*); Vol. 16, p.54 (1936), wherein vapor phase bromination of *p*-nitrotoluene is disclosed. The reaction was carried out in the presence of liquid bromine at 145-150°C. The yield of isolated product was 53-59 percent. HBr is obtained as byproduct and the overall atom efficiency vis-a-vis product formation is computed to be 28%.

Reference may be made to the same article above, wherein it is stated that the procedure of Brewster referred to above can be conveniently adopted by using artificial light in place of sunlight and that the light from two 300-watt tungsten lamps is satisfactory for the bromination of 300 g of *p*-nitrotoluene. The isolated yield of *p*-nitrobenzyl bromide is 60-70 per cent of the theoretical amount. Reference may again be made to the same article above wherein it is reported that although *p*-nitrobenzyl bromide is usually prepared by brominating *p*-nitrotoluene, it can also be prepared by treating *p*-nitrobenzyl alcohol with hydrobromic acid and by nitrating benzyl bromide. Reference may also be made to the paper entitled "A note on Bromination of p-nitrotoluene" by G. W. K. Cavill (J. of the Soc. Of Chem. Industry, 1946, 40, 124), wherein the bromination of *p*-nitrotoluene in the presence of liquid bromine and antimony tribromide is reported to yield *p*-nitrobenzyl bromide in 76% isolated yield. The reaction is undertaken at 120-130°C for 9-10 hrs. The bromine atom efficiency is computed to be 38%.

Reference may be made to U.S. Patent 6,740,253, which discloses the use of 2:1 sodium bromide-sodium bromate as atom efficient brominating agent. The reagent can be prepared cost-effectively from the 5:1 sodium bromide-sodium bromate intermediate obtained in the process of producing liquid bromine from sea bittern by the "cold process". Besides being a cost-effective reagent, the use of liquid bromine is avoided and higher bromine atom efficiencies are realized. The said brominating agent is disclosed for the preparation of *p*-nitrobenzyl bromide from *p*-nitrotoluene (S. Adimurthy et al. Green Chem., 2008, 10, 232). process has several advantages over the above stated prior art using liquid bromine but, even so, the conversion with respect to *p*-nitrotoluene is only 78% and with respect to Br it is 66%. Moreover, the work up of the reaction and purification of the product are tedious and there is a problem of management of organic waste containing largely dibromo impurity. Reference may also be made to the same paper, wherein it is stated that impurity formation can be reduced by taking large excess of *p*-nitrotoluene. However, the data provided is for the crude reaction mixture and nothing is mentioned about the work up of the reaction which would undoubtedly pose serious difficulties if the operations described in the paper are to be followed.

The present invention seeks to overcome the above drawbacks of the process of preparation of *p*-nitrobenzyl bromide from *p*-nitrotoluene employing the bromide/bromate couple of the prior art. The invention specifically discloses an improved process that is simple to execute and exhibits high yield of desired product with respect to both *p*-nitrotoluene and bromide/bromate reagent.

Adimurthy Subbarayappa et al. disclose disclose, in "An alternative method for the region-and stereoselective bromination of alkenes, alkynes, toluene, derevatives and ketones using a bromide/bromated couple" (Green chemistry, Royal Society of Chemistry, Cambridge, vol. 10, no.2, 1 January 2008, pages 232-237) a process for the preparation of p-nitrobenzyl bromide comprising loading a reaction vessel with 4-nitrotoluene, dichloromethane and brominating agent (2:1 Na Br/Na BrO₃), then adding to this mixture concentrated HCl-solution dropwise. Then, the layers are separated, the dichloromethane removed and the crude reaction product recristallysed from methanol. Further, recovered starting material from the mother liquor was recycled in second batch. In this process the separation of the product is difficult.

Reference may also be made to the paper entitled "A note on Bromination of p-nitrotoluene" by G. W. K. Cavill (J. of the Soc. Of Chem. Industry, 1946, 40, 124), wherein the bromination of p-nitrotoluene in the presence of liquid bromine and antimony tribromide is reported to yield *p*-nitrobenzyl bromide in 76% isolated yield. The reaction is undertaken at 120-130°C for 9-10 hrs. The bromine atom efficiency is computed to be 38%.

Reference may be made to U.S. Patent 6,740,253, which discloses the use of 2:1 sodium bromide-sodium bromate as atom efficient brominating agent. The reagent can be prepared cost-effectively from the 5:1 sodium bromide-sodium bromate intermediate obtained in the process of producing liquid bromine from sea bittern by the "cold process". Besides being a cost-effective reagent, the use of liquid bromine is avoided and higher bromine atom efficiencies are realized. The said brominating agent is disclosed for the preparation of *p*-nitrobenzyl bromide from *p*-nitrotoluene (S. Adimurthy et al. Green Chem., 2008, 10, 232). process has several advantages over the above stated prior art using liquid bromine but, even so, the conversion with respect to *p*-nitrotoluene is only 78% and with respect to Br it is 66%. Moreover, the work up of the reaction and purification of the product are tedious and there is a problem of management of organic waste containing largely dibromo impurity. Reference may also be made to the same paper, wherein it is stated that impurity formation can be reduced by taking large excess of *p*-nitrotoluene. However, the data provided is for the crude reaction mixture and nothing is mentioned about the work up of the reaction which would undoubtedly pose serious difficulties if the operations described in the paper are to be allowed.

The present invention seeks to overcome the above drawbacks of the process of preparation of *p*-nitrobenzyl bromide from *p*-nitrotoluene employing the bromide/bromate couple of the prior art. The invention specifically discloses an improved process that is simple to execute and exhibits high yield of desired product with respect to both *p*-nitrotoluene and bromide/bromate reagent.

Reference may be also made to the paper by Bell et. Al. (Bell, H. M.; Brown, H. C. J. Am. Chem. Soc. 1966, 88, 1473 pg) wherein a procedure is disclosed for the conversion of various benzyl bromides to substituted toluene derivative using $NaBH_4$ in dimethoxy ethane/water as a solvent. This is an important prior art in the context of the present invention which seeks to recycle over-brominated organic waste residue as one of the objects.

## OBJECTS OF THE INVENTION

The main object of the present invention is therefore to provide an improved process for the preparation of *p*-nitrobenzyl bromide from *p*-nitrotoluene using 2:1 bromide-bromate reagent which obviates the drawbacks of the prior art.

Another object of the present invention is to provide a process wherein the use of a single solvent for reaction, product isolation and purification eliminates the problem of contamination of one solvent with another and the process is made simple.

Yet another object of the present invention is to provide a process wherein *p*-nitrobenzyl bromide is selectively crystallized out from reaction mass through a chilling process which enables the mother liquor containing excess *p*-nitrotoluene and all of the solvent to be recycled directly.

Still another object of the present invention is to obtain product having ≥98% purity without recourse to re-crystallization.

A further object of the present invention is to provide a process which minimizes impurity formation and, consequently, leads to higher bromine atom efficiency.

Still another object of the present invention is to treat the residue remaining after distillation with sodium borohydride/ dimethoxy ethane (DME) to convert dibromo impurity back into *p*-nitrotoluene / *p*-nitrobenzyl bromide so that the mass can be once again recycled thereby converting waste into product, thereby circumventing the problem of organic waste disposal.

## SUMMARY OF THE INVENTION

The present invention provides an improved process for the preparation of *p*-nitrobenzyl bromide from *p*-nitrotoluene, wherein large excess of the reactant in carbon tetrachloride is treated with 2:1 bromide-bromate reagent and the product is isolated efficiently and with high purity through selective cold crystallization from reaction mass so as to allow the mother liquor to be recycled in a subsequent batch without any further work up. The cycles are continued so long as product of desired quality is obtained in adequate yield. Thereafter, the solvent and *p*-nitrotoluene are recovered from the mother liquor and the residue is treated with sodium borohydride to convert impurities back into reactant or product which is then recycled in the process thereby circumventing the problem of waste disposal and simultaneously yielding the desired product with >95 % yield with respect to *p*-nitrotoluene and >88 % bromine atom efficiency.

The major features of the present invention involve the following:

(i) selection of carbon tetrachloride as single organic solvent which enables the reaction to proceed in facile manner under reflux conditions and which also facilitates easy recovery of the desired product through chilling of the reaction mass.

(ii) taking two-fold excess of *p*-nitrotoluene to minimize over-bromination of product.

(iii) recycling of the mother liquor and washings from an earlier batch in a consequent batch so as to maintain same total *p*-nitrotoluene equivalent (i.e., unreacted *p*-nitrotoluene and *p*-nitrotoluene -based product/by-product).

(iv) taking brominating agent in subsequent batch to the same extent that p-nitrobenzyl bromide product was isolated in the previous batch so as to maintain consistency of stoichiometric ratio from batch-to-batch.

(v) avoiding cumbersome work up and instead, subjecting the mother liquor containing excess p-nitrotoluene and p-nitrobenzyl bromide to chilling at -15°C to -20°C temperature to crystallize out the latter selectively, thereafter allowing the mass to attain a temperature of -4°C, following which the mother liquor is separated out rapidly in a continuous centrifuge. The solid product is then  washed in the centrifuge with chilled (-4°C) carbon tetrachloride (2:1 v/w) collected towards the end of the reaction to attain >98% purity. -

(vi) minimizing carbon tetrachloride loss by carrying out the work up under chilled conditions in continuous centrifuge.

(vii) recovering carbon tetrachloride by ordinary distillation and p-nitrotoluene by vacuum distillation from mother liquor of last (8th) batch and treating the waste residue with NaBH$_4$ to convert dibromo impurity into p-nitrotoluene and p-nitrobenzyl bromide, which can be recycled to eliminate the problem of waste while increasing overall yield with respect to p-nitrotoluene and reagent.

Accordingly, the present invention provides an improved process for the preparation of p-nitrobenzyl bromide, wherein the steps comprising:

(i) dissolving 1.09 to 2.18 moles of a solid brominating reagent in water and adding into a reaction vessel;

(ii) adding a solution of p-nitrotoluene in carbon tetrachloride into the reaction vessel of step (i) to obtain a reaction mass;

(iii) raising the temperature of the reaction mass of step (ii) gradually to 80 to 90°C under continuous illumination and stirring;

(iv) adding a mineral acid in gradual amounts to the reaction mass of step (iii) as per the stoichiometric requirement;

(v) continuing the stirring under reflux conditions for additional 1.0 - 2.0 hours at the end of which the reaction mass becomes almost colorless;

(vi) switching from reflux mode as in step (v) to distillation mode and distilling out a part of the carbon tetrachloride which is chilled to a temperature in the range of -5 to -20 °C for washing of the centrifuged product as mentioned in (xi) below;

(vii) discontinuing the heating in step (vi) and allowing the reaction mass to cool to a temperature in the range of 35 to 40 °C;

(viii) separating the organic and aqueous layers from the cooled reaction mass of step (vii);

(ix) chilling the organic layer as obtained in step (viii) in a freezer to crystallize out p-nitrobenzyl bromide selectively;

(x) allowing the crystallized p-nitrobenzyl bromide of step (ix) to warm up to a temperature in the range of 0 to 10 °C followed by centrifuging to separate the crystals from the mother liquor;

(xi) washing the crystals as obtained in step (x) with the chilled carbon tetrachloride as obtained in step (vi) above to obtain purified p-nitrobenzyl bromide (having mp of 97-100 °C and GC purity of ca. 98%);

(xii) putting back mother liquor as obtained in step (x) and the washings as obtained in step (xi) into the reaction vessel for next cycle and topping up with required amount of p-nitrotoluene, brominating reagent and carbon tetrachloride;

(xiii) repeating the process of steps (iii) to (xii) over several cycles;

(xiv) recovering carbon tetrachloride and p-nitrotoluene successively from mother liquor and obtaining a residual mass;

(xv) dissolving the residual mass as obtained in step (xiv) in dimethoxy ethane and reacting under ambient condition with sodium borohydride to convert over-brominated products into a mixture of p-nitrotoluene and p-nitrobenzyl bromide;

(xvi) adding the mixture of p-nitrotoluene and p-nitrobenzyl bromide obtained in (xv) into the organic layer of step (viii).

## DETAILED DESCRIPTION OF THE INVENTION

The aim of the present invention is to improve upon the synthesis of p-nitrobenzyl bromide from p-nitrotoluene utilizing 2:1 mole ratio of Br$^-$:BrO$_3$$^-$ brominating reagent. Such brominating reagent is disclosed in the prior art to be produced cost-effectively from the 5:1 mole ratio of Br$^-$:BrO$_3$$^-$ obtained as intermediate in the cold process of bromine manufacture and further disclosed in the prior art to be useful for the synthesis of a large number of organo-bromine compounds including p-nitrobenzyl bromide (eq 1). More specifically, the utility highlighted is the ease of operation and the higher bromine atom efficiency. However, in the case of p-nitrobenzyl bromide preparation with 1.2 eqv of p-nitrotoluene with respect to brominating agent, the bromine atom efficiency reported employing the reagent is only 66% with generation of an organic waste which is difficult to dispose. Moreover, the process involves stripping off the solvent (methylene chloride/ethylene dichloride) followed by recrystallisation with hexane which is tedious. It has been indicated in the same prior art that higher ratio of p-nitrotoluene to brominating agent reduces impurity formation but no method of work up has been disclosed.

$$3C_7H_7NO_2 + 2\ NaBr + NaBrO_3 + 3/2\ H_2SO_4 \rightarrow 3C_7H_6NO_2Br + 3/2\ Na_2SO_4 + 3H_2O$$

**(equation 1)**

The present invention discloses an improved process for the synthesis of *p*-nitrobenzyl bromide, wherein:

(a) the reaction is carried out with large excess of *p*-nitrotoluene to achieve high yield and bromine atom efficiency of desired product;
(b) use of a single solvent throughout the process;
(c) isolation of desired product with high purity without recourse to recrystallization;
(d) recovery and reuse of excess *p*-nitrotoluene;
(e) elimination of organic waste.

The aforesaid improved process comprises the following major steps:

(i) dissolving solid brominating reagent in appropriate amount of water and adding into reaction vessel;
(ii) adding required amount of solution of *p*-nitrotoluene in carbon tetrachloride into the reaction vessel;
(iii) raising the temperature of the reaction mass gradually and carrying out the reaction under illumination;
(iv) adding in gradual amounts mineral acid as per the stoichiometry of equation 1;
(v) continuing the stirring under reflux condition for additional 1.0 to 2.0 hours at the end of which the reaction mass becomes almost colorless;
(vi) switching from reflux to distillation mode and collecting a part of the carbon tetrachloride for washing of the centrifuged product as mentioned in (xi) below;
(vii) discontinuing the heating and allowing the reaction mass to attain room temperature;
(viii) separating the organic and aqueous layers after cooling to room temperature;
(ix) chilling the organic layer in a freezer to crystallize out *p*-nitrobenzyl bromide selectively; also chilling the collected carbon tetrachloride ;
(x) removing from freezer and allowing the mass to warm up and thereafter centrifuging to separate the crystals from the mother liquor;
(xi) washing the crystals with the chilled carbon tetrachloride to obtain purified product (having mp of 97-100°C and GC purity of ca. 98%);
(xii) putting back mother liquor and the washings into the reaction vessel for next cycle and topping up with required amount of *p*-nitrotoluene, brominating reagent and carbon tetrachloride;
(xiii) repeating the process of steps (iii) to (xii) and continuing for a total of seven recycles, i.e., 8 batches in all;
(xiv) recovering the carbon tetrachloride from mother liquor by ordinary distillation;
(xv) recovering *p*-nitrotoluene in the residue by vacuum distillation;
(xvi) dissolving the residual mass in dimethoxy ethane and reacting with sodium borohydride to convert over-brominated products into a mixture of *p*-nitrotoluene and *p*-nitrobenzyl bromide;
(xvii) adding the mixture of *p*-nitrotoluene and *p*-nitrobenzyl bromide obtained in (xv) into the organic layer of step (viii);
(xviii) recovering the DME by distillation and then leaching out water soluble impurities from the residue and utilize the resultant product in recycle steps.

In an embodiment of the present invention, the active bromine content in the solid brominating reagent (2:1 mole ratio of $Br^-/BrO_3^-$) used in step (i) above is 42.0% (w/w) while the active Br concentration in the aqueous solution is 2.4 M.
In another embodiment of the present invention, commercial grade *p*-nitrotoluene is used and its initial concentration in carbon tetrachloride is in the range of 0.15 to 0.35 kg/L.
In still another embodiment of the present invention, 0.388 kg of brominating reagent containing 2.189 moles of active Br is dissolved in 0.9 L water and used in the form of aqueous brominating reagent in step (i) above.
In yet still another embodiment of the present invention, the mole equivalent of p-nitrotoluene to moles of active bromine is in the range of 1.0 to 5.0.
In still another embodiment of the present invention, the reaction vessel used in step (i) above is a jacketed 10 L capacity 3-neck round bottom glass reactor having bottom discharge and equipped with reflux-cum-distillation assembly, mechanical stirrer, side arm liquid addition facility and three externally fitted 100 W tungsten filament lamps. In yet another embodiment of the present invention, 6.57 mole of commercial grade *p*-nitrotoluene is used in step (ii) above.
In another embodiment of the present invention, the brominating reagent used has a bromide ion to bromate ion mole ratio in the range of 1.8:1 to 2.1:1 and its concentration (total Br) is in the range of 2 to 3 molar in water.

In still another embodiment of the present invention, 4.8 L of commercial grade carbon tetrachloride is used in step (ii) above.

In yet another embodiment of the present invention, 1 to 4 light bulbs of 40-250 W power rating are used for illumination. In a further embodiment of the present invention, the contents of the reactor are heated slowly with a water condenser to maintain a temperature of $90 \pm 1°C$ in step (iii) above.

In still another embodiment of the present invention, 1.04 equivalents of 15% (w/v) sulfuric acid (w.r.t. Br equivalent taken) are added to the hot reaction mixture in step (iv) above, under continuous stirring.

In another embodiment of the present invention, 0.5 to 2.5 molar sulphuric acid is added over a period of 0.5 to 3 hours in step (iv).

In yet another embodiment of the present invention, the reflux conditions in step (v) above are maintained for an additional 2 hours under constant stirring.

In still another embodiment of the present invention, after the reaction became colorless

in step (vi) above, the reflux is switched to distillation mode and 0.6 L carbon tetrachloride is collected for washing the centrifuged product as mentioned in step (xi) above.

In still another embodiment of the present invention, the amount of carbon tetrachloride distilled out in step (vi) is in the range of 10-20% of the total amount taken which is chilled to a temperature of -5 to -20 °C, preferably to 10°C.

In yet another embodiment of the present invention, the heating in step (vii) above is discontinued and the reaction mixture is allowed to attain room temperature.

In still another embodiment of the present invention, in step (viii) above, the aqueous and organic phases are separated.

In yet another embodiment of the present invention, the organic phase in step (ix) above is kept for chilling at -20 to -15°C for 12.0 hrs.

In still another embodiment of the present invention, the carbon tetrachloride distilled off in step (vi) above, is also kept for chilling.

In yet another embodiment of the present invention, the mass in step (x) above is allowed to attain the room temperature and thereafter the product is isolated by centrifuging at -4°C in a continuous centrifuge.

In yet another embodiment of the present invention, the centrifuged mass in step (xi) above is washed twice with chilled 0.6L carbon tetrachloride collected by distillation in step (vi) above.

In another embodiment of the present invention, the crystals having a melting point in the range of 97 -100 °C and GC purity of ca. 98 % are collected in step (xi).

In yet another embodiment of the present invention, the mother liquor obtained in step (x) above and the washings obtained in step (xi) above are put back into the reaction vessel for next cycle in step (xii) above.

In still another embodiment of the present invention, the reaction vessel in step (xii) above is topped up with 1.68 moles of commercial grade p-nitrotoluene, 0.332 kg of brominating reagent containing 1.872 moles of active Br; and 6.17 moles of carbon tetrachloride.

In yet another embodiment of the present invention, the process of steps (iii) to (xii) are repeated and continued for a total of seven recycles, i.e., 8 batches in all to give 2.947 kg of p-nitrobenzyl bromide with average of 98.2% purity.

In still another embodiment of the present invention, the repetition of cycles as mentioned in step (xiii) is preferably done for a total of seven cycles so long as the average purity of product over all the accumulated cycles is $\geq 98\%$.

In a further embodiment of the present invention, the carbon tetrachloride in step (xiv) above, is recovered from mother liquor by ordinary distillation.

In another embodiment of the present invention, carbon tetrachloride is recovered from spent mother liquor by distillation and p-nitrotoluene by vacuum distillation at 10-40 mm of Hg and distillation temperature in the range of 130-155°C.

In still another embodiment of the present invention, 3.75 moles of p-nitrotoluene is recovered in step (xv) above, from the residue by vacuum distillation.

In yet another embodiment of the present invention, the ratio of residual mass to dimethoxy ethane in step (xvi) is in the range of 1:2 to 1:5 w/v.

In stilt another embodiment of the present invention, 0.323 kg of black residue enriched with dibromo impurity is obtained in step (xv) above, which is used in step (xvi).

In yet another embodiment of the present invention, 0.5 to 4.0 mole equivalents of sodium borohydride is taken together with the residual mass in a solvent followed by adding a 2.5:1 v/v solution of dimethoxy ethane : water gradually under stirring in step (xvi).

In another embodiment of the present invention, in step (xvi) above, 0.052 kg of black residue enriched with dibromo impurity is dissolved in 0.012 L DME to which is added 0.027 kg of $NaBH_4$ at room temperature and then a mixture of 0.012 1 DME + 0.005 L $H_2O$ is added drop wise and allowed to stir for 2 hours.

In yet another embodiment of the present invention, the DME in step (xvii) above is distilled and the water soluble impurities are leached out. Further, the resultant product is utilized in recycle steps.

In another embodiment of the present invention, in step (xvii) the mole ratio of *p*-nitrotoluene to *p*-nitrobenzyl bromide is 2:1 to 1:3.

In still another embodiment of the present invention, the yield of *p*-nitrobenzyl bromide is 90-98% with respect to *p*-nitrotoluene and the bromine atom efficiency is 88-92 %.

In yet another embodiment of the present invention, the average purity of *p*-nitrobenzyl bromide is ≥ 98% (GC area %).

In still another embodiment of the present invention, the process is free of organic waste.

*The following examples are given by way of illustration and therefore should not be construed to limit the scope of the present invention.*

### EXAMPLE 1

60 g (437.9 mmoles) of *p*-nitrotoluene was dissolved in 324 mL of carbon tetrachloride and added into a 1.0 L capacity glass reactor fitted with addition funnel and reflux condenser/distillation assembly. 90 mL of brominating agent containing 145.9 mmole active Br was added so that the *p*-nitrotoluene to *p*-nitrobenzyl bromide ratio is ≥ 2:1 throughout the reaction. The contents were heated to reflux temperature and illuminated externally with a 100 W tungsten filament bulb. 7.3 g of 98% $H_2SO_4$ (72.5 mmole) in 30 mL of water was taken in the addition funnel and the acid was added over a period of 2 h. (Caution: If the reaction mass develops brown color stop the addition and allow color to disappear before continuing addition of acid.) Reflux conditions were maintained for an additional 1 h. The reaction mass was allowed to cool to room temperature and the organic and aqueous layers were then separated. The organic phase was poured into a beaker and kept in the freezer compartment of a refrigerator overnight. Crystal formation was observed. The mass was allowed to warm up to 0 - 4 °C and filtered under vacuum. The crystals which collected in the funnel were washed with chilled carbon tetrachloride. The collected *p*-nitrobenzyl bromide was kept for crystallisation in 4.8 w/v carbon tetrachloride. The yield of collected *p*-nitrobenzyl bromide was 15.00 g (69.44 mmol). The mother liquor along with washings was put back into the glass reactor. Fresh 18.8 g (137.2) mmol of *p*-nitrotoluene were added and brominating reagent was taken at the mole ratio of 1.1-1.2 with respect to fresh *p*-nitrotoluene added. The reaction was repeated to obtain additional amount of *p*-nitrobenzyl bromide, while the mother liquor & washings were once again recycled. Four such recycled batches were carried out as shown in Table 1 below. Carbon tetrachloride (density =1.5842 g/cm$^3$) was recovered from the mother liquor for reuse.

**Table 1**

| Sr. No. | *p*-nitrotoluene wt.(g)/ (mmole) | carbon tetrachloride (ml) | Br/mole | 98 % $H_2SO_4$ wt/ (mmole) | *p*-nitrobenzyl bromide wt/(mmole) |
|---|---|---|---|---|---|
| 1. | 60.00 (437.9) | 324 | 145.9 | 7.3 g (72.5) | 15.00 (69.44) |
| 2. | 18.80 (137.2) | Recycled mother liquor | 101.3 | 5.0 g (50.0) | 15.02 (69.53) |
| 3. | 18.80 (137.2) | Recycled mother liquor | 101.2 | 5.0 g (50.0) | 16.91 (78.28) |
| 4. | 10 (73.00) | Recycled M.L. + M.L. from recrystallization | 44.1 | 3.58 g (35.8) | 16.95 (78.47) |
| 5. | 10 (73.00) | Recycled M.L. + M.L. from recrystallization | 73.7 | 3.58 g (35.8) | 19.3 (89.35) |
| Total | 117.6 (858.3) | | 466.2 | 24.31 g (243.1) | 83.1 (385.0) |

The above example teaches us that *p*-nitrobenzyl bromide can be selectively crystallized out in the reaction solvent itself at sub-zero temperature even in presence of large excess of *p*-nitrotoluene. This enables easy recycling of the mother liquor to realize a bromine atom efficiency of nearly 83%. The yield of product with respect to *p*-nitrotoluene consumed is also nearly quantitative but it is only 45% in the absence of recovery of *p*-nitrotoluene lost in the final mother liquor.

### EXAMPLE 2

0.9 kg (6.57 mole) of *p*-nitrotoluene was dissolved in 4.8 L of carbon tetrachloride and added into a 10L capacity glass reactor fitted with addition funnel and reflux condenser/distillation assembly. 1.09 L of brominating agent containing 2.18 mole active Br was added so that the *p*-nitrotoluene to *p*-nitrobenzyl bromide ratio is $\geq$ 2:1 throughout the reaction. The contents were heated to reflux temperature and illuminated externally with 3 x 100 W tungsten filament bulbs. 112 g of 98% $H_2SO_4$ (1.12 mole) in 0.6 L of water was taken in the addition funnel and the acid was added over a period of 2 h. (Caution: If the reaction mass develops brown color stop the addition and allow color to disappear before continuing addition of acid.) Reflux conditions were maintained for an additional 1 h. The reaction mass was allowed to cool to ca. 40°C temperature and the contents discharged into a separating column. The organic phase was poured into a suitable wide mouth glass vessel and kept for crystallization in a deep freezer after inserting 3 broomsticks to promote crystallization. The organic phase attained a temperature of - 17.5$\pm$2.5 °C and was removed after 12 hours. Crystal formation was seen. The contents were allowed to warm up to 5$\pm$2.5° C to re-dissolve *p*-nitrotoluene which had co-crystallized with the product. The lumps of crystals were broken up and the contents centrifuged in a continuous centrifuge which operation required barely a couple of minutes (note that the mother liquor warmed up to around 7.5$\pm$2.5 °C during the centrifuging). The collected *p*-nitrobenzyl bromide weighed 0.264 kg (1.22 moles) and exhibited 95% (GC area %) purity. The mother liquor was put back into the glass reactor. Fresh 0.167 kg 1.22 moles of *p*-nitrotoluene, equivalent to the amount of *p*-nitrobenzyl bromide crystallized out was added and brominating reagent was taken at the mole ratio as shown in Table 2 below. The reaction was repeated in the same manner as above but towards the end of the  reaction 0.6 L of carbon tetrachloride was collected by changing over from reflux mode to distillation mode and kept for chilling. Cold crystallization was repeated to obtain *p*-nitrobenzyl bromide from the reaction mass. The crude *p*-nitrobenzyl bromide was washed in the centrifuge with the chilled carbon tetrachloride and centrifuged. The mother liquor and washings were once again recycled. Seven such recycled batches were carried out and the data on *p*-nitrobenzyl bromide yield and purity for each cycle and also the total yield and average purity are provided in table2 below. Carbon tetrachloride was recovered from the final mother liquor for reuse.

**Table 2**

| Sr. No. | *p*-nitrotoluene wt.Kg/(mole) | Carbon tetrachloride (L) | Br/mole | 98 % $H_2SO_4$ wt/ (mole) | *p*-nitrobenzyl bromide wt/ (mole) | Purity GC area % |
|---|---|---|---|---|---|---|
| 1. | 0.900 (6.57) | 4.8 | 2.189 | 0.112.0 Kg (1.10) | 0.264 (1.22) | 95 |
| 2. | 0.167 (1.22) | Recycled Mother liquor | 1.437 | 0.75.0 Kg (0.73) | 0.290 (1.34) | 97.8 |
| 3. | 0.184 (1.34) | Recycled Mother liquor | 1.578 | 0.81 Kg (0.79) | 0.278 (1.28) | 98.9 |
| 4. | 0.176 (1.28) | Recycled Mother liquor | 1.42 | 0.74 Kg (0.72) | 0.300 (1.38) | 100 |
| 5. | 0.190 (1.38) | Recycled Mother liquor | 1.54 | 0.80 Kg (0.78) | 0.290 (1.34) | 100 |
| 6. | 0.184 (1.34) | Recycled Mother liquor | 1.49 | 0.77 Kg (0.75) | 0.319 (1.47) | 100 |
| 7. | 0.202 (1.47) | Recycled Mother liquor | 1.63 | 0.84 Kg (0.82) | 0.310 (1.43) | 100 |
| 8. | 0.196 (1.43) | Recycled Mother liquor | 1.59 | 0.82 Kg (0.80) | 0.310 (1.43) | 95.12 |
| Total | 2.201 (16.03) | 4.5 L Carbon tetrachloride recovered Mother liquor | 12.86 | 0.665 Kg (6.49) | 2.361 (10.93) | 98.4% average purity |

This example teaches us that the laboratory process is easily amenable to scale up and that by carrying out the operations more efficiently and recycling the mother liquor seven times instead of only four times, the yield of p-nitrobenzyl bromide with respect to *p*-nitrotoluene was increased to 68.2% while bromine atom efficiency was 84.8%. The example further teaches us that mere washing of the crystals with chilled carbon tetrachloride is sufficient to yield a product of high purity.

**EXAMPLE 3**

The experiment of Example 2 was repeated and the stoichiometry of reagents for each cycle as also yield and purity of product are shown in Table 3 below. After the 8th cycle, carbon tetrachloride was recovered from the mother liquor as before and the residue was then subjected to vacuum distillation. For this purpose, the residue was first melted and purged with nitrogen. Thereafter, the temperature was raised while applying vacuum and the *p*-nitrotoluene distilled over at the reduced pressure [temperature: 140 °C (initial)-155 °C (final); pressure: 40 mm (initial) -10 mm (final) Hg]. The amount of *p*-nitrotoluene recovered was 0.514 kg (3.75 moles).
Note: small amounts of *p*-nitrobenzyl bromide were also found to distill over. The recovered *p*-nitrotoluene amount was deducted from the total amount of *p*-nitrotoluene charged for the purpose of calculating conversion with respect to *p*-nitrotoluene. As calculated based on the data of Table 3 below, the yield of *p*-nitrobenzyl bromide was 91.7% with respect to *p*-nitrotoluene consumed while the bromine atom efficiency was 87.3%.

**Table 3**

| Batch No. | *p*-nitrotoluene Kg/(mol) | Carbon tetrachloride (L) | Br/mole | 98% $H_2SO_4$ Kg (mol) | *p*-nitrobenzyl bromide Kg/ (mol) | Purity (GC area %) |
|---|---|---|---|---|---|---|
| 1 | 0.900 (6.56) | 4.8 | 2.189 | 0.114 (1.14) | 0.364 (1.68) | 97.1 |
| 2 | 0.231 (1.68) | Recycled mother liquor | 1.87 | 0.107 (1.07) | 0.368 (1.7) | 95.7 |
| 3 | 0.233 (1.70) | Recycled Mother liquor | 1.89 | 0.107 (1.07) | 0.369 (1.7) | 99.5 |
| 4 | 0.234 (1.70) | Recycled Mother liquor | 1.9 | 0.107 (1.07) | 0.373 (1.72) | 100 |
| 5 | 0.236 (1.72) | Recycled Mother liquor | 1.92 | 0.095 (0.95) | 0.371 (1.71) | 98.5 |
| 6 | 0.235 (1.71) | Recycled Mother liquor | 1.91 | 0.107 (1.07) | 0.411 (1.9) | 96.3 |
| 7 | 0.260 (1.90) | Recycled Mother liquor | 2.11 | 0.107 (1.07) | 0.360 (1.66) | 100 |
| 8 | 0.228 (1.66) | Recycled Mother liquor | 1.85 | 0.107 (1.07) | 0.330 (1.52) | 98.8 |
| Total/Av | 2.559 (18.63) | 4.5 L Carbon tetrachloride recovered | 15.63 | 0.851 (8.51) | 2.947 (13.59) | 98.2 |

Examples 3 teaches us that it is simple to recover *p*-nitrotoluene from the residue of the final cycle and that this makes it feasible to carry out the reaction with large excess of *p*-nitrotoluene to realize the benefit of lower impurity formation.

**EXAMPLE 4**

The dark residue weighing 323 g recovered in Example 3 after vacuum distillation of *p*-nitrotoluene was analyzed by GC-MS and NMR and found to comprise 71.1% dibromo ($NO_2$-Ar-$CHBr_2$) impurity and 26.9% *p*-nitrobenzyl bromide as the main constituents. 5.23 g of this residue was dissolved in 12.5 ml of dimethoxy ethane (DME). 2.69 g of $NaBH_4$ was added next. A solution made from 12.5 ml DME and 5 ml water was then added gradually over 1 h under stirring at room temperature. The DME was then distilled at reduced pressure to yield a solid, which was washed with water to give 2.1 g of pale yellow solid whose GC indicated that the dibromo impurity in residue was converted into *p*-nitrotoluene and *p*-nitrobenzyl bromide, giving a composition comprising 60.5% *p*-nitrotoluene and 39.5% p-nitrobenzyl bromide based on GC area %. It would be evident that the converted residue having nearly 2:1 ratio of *p*-nitrotoluene: *p*-nitrobenzyl bromide can be recycled. This way the conversion increased to 96% with respect to *p*-nitrotoluene consumed and the bromine atom efficiency increased to 90%.

**EXAMPLE** 5

Then aqueous effluent from 8th batch of Example 3 was collected and analyzed. The data is provided in Table 4 below. It can be seen that the aqueous effluent has low levels of organic matter and unreacted bromide.

**Table 4. Data showing details of aqueous effluent from 8th batch of Example 3**

| Parameters | Value | Units |
|---|---|---|
| Volume of effluent | 1.63 | Litres |
| pH | 1.75 | |
| TDS | 161.9 | mg/L |
| Density | 1.144 | gm/cc at 32.3°C |
| DO | 2.93 | mg/L |
| TOC | 91.2 | mg/L |
| COD | 503.4 | mg/L |
| Chloride | 4.7 | % |
| BOD | Nil | mg/L |
| Bromide | 0.6 | g/L |

**The main advantages of the present invention are:**

1. By using a single solvent for reaction, product isolation and purification, the problem of contamination of one solvent with another is eliminated and the process made simple.

2. *p*-nitrobenzyl bromide is selectively crystallized out from reaction mass through a chilling process, which enables the mother liquor containing excess *p*-nitrotoluene and all of the solvent to be recycled directly.

3. *p*-nitrobenzyl bromide having >98% purity is obtained merely by washing the product with chilled carbon tetrachloride.

4. The present process minimizes solvent losses.

5. The process minimizes impurity formation and consequently, leads to higher bromine atom efficiency.

6. Excess *p*-nitrotoluene required to minimize impurity formation is easily recovered.

7. Waste organic residue comprising largely dibromo impurity is converted back into *p*-nitrotoluene/*p*-nitrobenzyl bromide, which not only increases the yield with respect to *p*-nitrotoluene but also circumvents the problem of organic waste disposal.

8. The aqueous effluent contains minimum organic load.

**Claims**

1.   A process for the preparation of *p*-nitrobenzyl bromide, wherein the steps comprising:

(i) adding a solution of *p*-nitrotoluene in carbon tetrachloride having concentration in the range of 0.15 to 0.35 kg/L into a reaction vessel;

(ii) dissolving 2:1 alkali bromide/alkali bromate solid brominating reagent in water to a concentration of 2-3 M active Br and adding into the reaction vessel of step (i) maintaining substrate to active bromine (in reagent) ratio in the range of 1 : 1 to 5: 1 ;

(iii) raising the temperature of the reaction mass of step (ii) gradually from ambient to 50-60°C under continuous illumination;

(iv) adding stoichiometric amount of 0.5-2.5 M mineral acid such as sulphuric acid and hydrochloric acid into the reaction mass of step (iii) over a period of 1-3 hours under continuous stirring;

(v) continuing the stirring under reflux conditions for additional 1.0 - 2.0 hours at the end of which the reaction mass becomes almost colorless;

(vi) switching from reflux mode of step (v) to distillation mode and distilling out 10-15% of the carbon tetrachloride taken which is thereafter chilled to a temperature in the range of -5 to -20°C for washing of the centrifuged product as mentioned in (xi) below;

(vii) discontinuing the heating after the distillation of step (vi) and allowing the reaction mass to cool to 35-40°C;

(viii) separating the organic and aqueous layers from the cooled reaction mass of step (vii);

(ix) chilling the organic layer obtained in step (viii) in a freezer to crystallize out *p*-nitrobenzyl bromide selectively in the temperature range of -5 to - 20°C;

(x) allowing the crystallized *p*-nitrobenzyl bromide of step (ix) to warm up to a temperature in the range of 0-10°C followed by centrifuging to separate the crystals from the mother liquor;

(xi) washing the crystals obtained in step (x) with the chilled carbon tetrachloride obtained in step (vi) to obtain purified *p*-nitrobenzyl bromide having mp of 97-100° C and GC purity of ca. 98%;

(xii) putting back mother liquor of step (x) and washings of step (xi) into the reaction vessel for next cycle and topping up with required amounts of *p*-nitrotoluene, brominating reagent and carbon tetrachloride;

(xiii) repeating the process of steps (iii) to (xii) over several cycles;

(xiv) distilling out under atmospheric pressure carbon tetrachloride in the spent organic layer after completion of recycles and thereafter distilling out *p*-nitrotoluene at under 130-155° C under a reduced pressure of 33900-135000 Pa (10-40 mm of Hg).

(xv) dissolving the residual mass (RM) of step (xiv) in dimethoxy ethane (DME) in the RM:DME ratio of 1:2 to 1:5 (w/v) and thereafter treating the mass under stirring at room temperature with sodium borohydride ($NaBH_4$) in the $RM:NaBH_4$ weight ratio of 3:1 to 1.5:1 to convert over-brominated products into a mixture of *p*-nitrotoluene and *p*-nitrobenzyl bromide;

(xvi) adding the mixture of *p*-nitrotoluene and *p*-nitrobenzyl bromide obtained in (xv) into the organic layer of step (viii) prior to continuing the operations of steps (ix) to (xv) and thereby eliminating organic waste.

2. A process as claimed in claim 1, wherein the reaction temperature in step (iii) is preferably raised to 50°C.

3. A process as claimed in claim 1, wherein 1 to 4 light bulbs of 40-250 W power rating are used for illumination in step (iii), when the glass reactor vessel size was 1 to 10 L.

4. A process as claimed in claim 1, wherein seven to ten recycles of mother liquor are undertaken before subjecting the spent mother liquor to the operations of steps (xiii to xvi).

5. A process as claimed in claim 1, wherein dimethoxy ethane is recovered by distillation and the product washed with water as part of the operation sin step (xv).

6. A process as claimed in claim 1, wherein in step (xvi) the mole ratio of *p*-nitrotoluene to *p*-nitrobenzyl bromide after mixing of the organic layers is 2:1 to 1:3.

7. A process as claimed in any preceding claims, wherein the yield of *p*-nitrobenzyl bromide is 90-98% with respect to *p*-nitrotoluene and the bromine atom efficiency is 88-92 %.

8. A process as claimed in any preceding claims, wherein the average purity of *p*-nitrobenzyl bromide is > 98% (GC area %).

**Patentansprüche**

1. Verfahren zur Herstellung von *p*-Nitrobenzylbromid, wobei die Schritte Folgendes umfassen:

(i) Zugeben einer Lösung von *p*-Nitrotoluol in Tetrachlorkohlenstoff mit einer Konzentration im Bereich von 0,15 bis 0,35 kg/L in ein Reaktionsgefäß;

(ii) Auflösen eines festen Bromierunaamittels mit 2:1 Alkalibromid/Alkalibromat in Wasser auf eine Konzentration von 2-3 M aktivem Br und Zugeben in das Reaktionsgefäß von Schritt (i), sodass ein Verhältnis von Substrat zu aktivem Brom (im Reagens) in dem Bereich von 1:1 bis 5:1 erhalten wird;

(iii) stufenweises Erhöhen der Temperatur der Reaktionsmasse aus Schritt (ii) von Umgebungstemperatur auf 50-60°C unter kontinuierlicher Belichtung;

(iv) Zugabe einer stöchiometrischen Menge an 0,5-2,5 M Mineralsäure, wie zum Beispiel Schwefelsäure und Salzsäure, in die Reaktionsmasse von Schritt (iii) über einen Zeitraum von 1-3 Stunden unter kontinuierlichem Rühren;

(v) Fortsetzen des Rührens unter Rückflussbedingungen für zusätzliche 1,0 bis 2,0 Stunden, wobei am Ende dieser Zeit die Reaktionsmasse nahezu farblos wird;

(vi) Umschalten vom Rückflussmodus von Schritt (v) in den Destilliermodus und Abdestillieren von 10-15% des verwendeten Tetrachlorkohlenstoffs, wobei hernach auf eine Temperatur im Bereich von -5 bis -20°C abgekühlt wird, um das zentrifugierte Produkt zu waschen, wie in (xi) unten erwähnt,

(vii) Unterbrechen des Erhitzens nach der Destillation von Schritt (vi) und Abkühlenlassen der Reaktionsmasse auf 35-40°C;

(viii) Abtrennen der organischen und wässrigen Phasen aus der abgekühlten Reaktionsmasse aus Schritt (vii);

(ix) Kühlen der in (viii) erhaltenen organischen Phase in einem Kühlschrank, um p-Nitrobenzylbromid selektiv im Temperaturbereich von -5 bis -20°C auszukristallisieren;

(x) Erwärmenlassen des kristallisierten p-Nitrobenzylbromids aus Schritt (ix) auf eine Temperatur im Bereich von 0-10°C, gefolgt von Zentrifugieren, um die Kristalle von der Mutterlauge abzutrennen;

(xi) Waschen der in Schritt (x) erhaltenen Kristalle mit dem in Schritt (vi) erhaltenen abgekühlten Tetrachlorkohlenstoff unter Erhalt von gereinigtem p-Nitrobenzylbromid mit einem Smp. von 97-100°C und einer GC-Reinheit von ca. 98%;

(xii) Zurückübertragen der Mutterlauge von Schritt (x) und der Waschungen von Schritt (xi) in das Reaktionsgefäß für den nächsten Zyklus, und Nachfüllen mit den erforderlichen Mengen an p-Nitrotoluol, Bromierungsmittel und Tetrachlorkohlenstoff;

(xiii) Wiederholen des Verfahrens nach Schritten (iii) bis (xii) über mehrere Zyklen hinweg;

(xiv) Abdestillieren von Tetrachlorkohlenstoff in der verbrauchten organischen Phase unter atmosphärischem Druck nach Vervollständigung der Zyklen und anschließend Abdestillieren von p-Nitrotoluol bei 130-155°C unter einem verringerten Druck von 33900-135000 Pa (10-40 mg Hg);

(xv) Auflösen der verbleibenden Masse (RM) von Schritt (xiv) in Dimethoxyethan (DME) in einem Verhältnis von RM:DME von 1:2 bis 1:5 (G/V) und anschließend Behandeln der Masse mit Natriumborhydrid (NaBH$_4$) in einem Gewichtsverhältnis von RM:NaBH$_4$ von 3:1 bis 1,5:1 unter Rühren bei Raumtemperatur, unter Umwandlung von überbromierten Produkten in eine Mischung von p-Nitrotoluol und p-Nitrobenzylbromid;

(xvi) Zugeben der Mischung von p-Nitrotoluol und p-Nitrobenzylbromid, erhalten in (xv), zu der organischen Phase von Schritt (viii) vor dem Fortführen der Arbeitsschritte gemäß Schritten (ix) bis (xv), und dadurch Eliminieren von organischem Abfall.

2. Verfahren gemäß Anspruch 1, wobei die Reaktionstemperatur in Schritt (iii) vorzugsweise auf 50°C abgehoben wird.

3. Verfahren gemäß Anspruch 1, wobei 1 bis 4 Glühbirnen von 40-250 W Leistung zur Belichtung in Schritt (iii) verwendet werden, wobei die Größe des Glasreaktionsgefäßes 1-10 L beträgt.

4. Verfahren gemäß Anspruch 1, wobei 7 bis 10 Wiederverwendungen der Mutterlauge vorgenommen werden, bevor die verbrauchte Mutterlauge den Arbeitsgängen der Schritte (xiii bis xvi) unterworfen wird.

5. Verfahren gemäß Anspruch 1, wobei Dimethoxyethan durch Destillation wiedergewonnen wird, und das Produkt mit Wasser als Teil der Arbeitsgänge in Schritt (xv) gewaschen wird.

6. Verfahren gemäß Anspruch 1, wobei in Schritt (xvi) das Molverhältnis von p-Nitrotoluol zu p-Nitrobenzylbromid nach Mischen der organischen Phasen 2:1 bis 1:3 beträgt.

7. Verfahren gemäß einem der vorstehenden Ansprüche, wobei die Ausbeute an p-Nitrobenzylbromid 90-98% bezüglich p-Nitrotoluol beträgt, und die Brom-Atomökonomie 88-92% beträgt.

8. Verfahren gemäß einem der vorstehenden Absprüche, wobei die durchschnittliche Reinheit von p-Nitrobenzylbromid >98% (GC-Flächen-%) beträgt.

## Revendications

1. Procédé de préparation de bromure de p-nitro-benzyle, comprenant les étapes suivantes :

(i) l'ajout d'une solution de p-nitrotoluène dans le tétrachlorure de carbone, ayant une concentration dans la plage allant de 0,15 kg/l et 0,35 kg/l, à un récipient réactionnel ;

(ii) la dissolution d'un réactif solide de bromation à base de bromure d'alcalin/bromate d'alcalin 2/1 dans de l'eau jusqu'à l'obtention d'une concentration de Br actif de 2 M à 3 M et son ajout au récipient réactionnel de l'étape (i) en maintenant le rapport substrat/bromure actif (dans le réactif) dans la plage allant de 1/1 à 5/1 ;

(iii) l'augmentation progressive de la température de la masse réactionnelle de l'étape (ii) à partir de la température ambiante jusqu'à une température de 50 °C à 60 °C sous éclairage continu ;

(iv) l'ajout d'une quantité stoechiométrique d'un acide minérale à 0,5-2,5 M, tel que l'acide sulfurique et l'acide chlorhydrique, à la masse réactionnelle de l'étape (iii) une période de 1 à 3 heures sous agitation continue ;

(v) la poursuite de l'agitation dans des conditions de reflux pendant 1,0 à 2,0 heures supplémentaires, après quoi la masse réactionnelle devient pratiquement incolore ;

(vi) le passage du mode reflux de l'étape (v) à un mode distillation et la distillation de 10 % à 15 % du tétrachlorure de carbone pris, qui est ensuite  refroidie jusqu'à une température située dans la plage allant de -5 °C à -20 °C pour laver le produit centrifugé comme décrit dans l'étape (xi) ci-dessous ;

(vii) l'arrêt du chauffage après la distillation de l'étape (vi) et le fait de laisser la masse réactionnelle refroidir jusqu'à une température de 35 °C à 40 °C ;

(viii) la séparation de la phase organique et de la phase aqueuse de la masse réactionnelle refroidie de l'étape (vii) ;

(ix) le refroidissement de la phase organique obtenue dans l'étape (viii) dans un congélateur pour que le bromure de p-nitrobenzyle cristallise sélectivement dans la plage de températures allant de -5 °C à -20 °C ;

(x) le fait de laisser le bromure de $p$-nitrobenzyle cristallisé obtenu dans l'étape (ix) se réchauffer jusqu'à une température située dans la plage allant de 0 °C à 10 °C, puis la séparation des cristaux de la liqueur mère par centrifugation ;

(xi) le lavage des cristaux obtenus dans l'étape (x) avec le tétrachlorure de carbone refroidi obtenu dans l'étape (vi) pour obtenir du bromure de $p$-nitro-benzyle purifié présentant un pf de 97-100 °C et une pureté par CG d'environ 98 % ;

(xii) la réintroduction de la liqueur mère de l'étape (x) et des lavages de l'étape (xi) dans le récipient réactionnel pour le cycle suivant et son remplissage avec les quantités requises de $p$-nitro-toluène, de réactif de bromation et de tétrachlorure de carbone ;

(xiii) la répétition des étapes (iii) à (xii) du procédé pendant plusieurs cycles ;

(xiv) la distillation sous pression atmosphérique du tétrachlorure de carbone se trouvant dans la phase organique usagée à la fin des recyclages, puis la distillation du $p$-nitrotoluène à une température de 130 °C à 155 °C sous une pression réduite de 33 900 Pa à 135 000 Pa (10 à 40 mm de Hg) ;

(xv) la dissolution de la masse résiduelle (RM) de l'étape (xiv) dans du diméthoxyéthane (DME) selon un rapport RM/DME de 1/2 à 1/5 (poids/volume), puis le traitement de la masse sous agitation à température ambiante avec du borohydrure de sodium ($NaBH_4$) selon un rapport pondéral RM/$NaBH_4$ de 3/1 à 1,5/1 afin de convertir les produits sur-bromés en un mélange contenant du $p$-nitrotoluène et du bromure de $p$-nitro-benzyle ;

(xvi) l'ajout du mélange contenant du $p$-nitro-toluène et du bromure de $p$-nitrobenzyle obtenu dans l'étape (xv) à la phase organique de l'étape (viii) avant de poursuivre les opérations des étapes (ix) à (xv) et ainsi d'éliminer les déchets organiques.

2. Procédé selon la revendication 1, dans lequel la température réactionnelle dans l'étape (iii) est augmentée de préférence jusqu'à 50 °C.

3. Procédé selon la revendication 1, dans lequel 1 à 4 ampoules d'une puissance nominale de 40 W à 250 W sont utilisées pour l'éclairage dans l'étape (iii), quand la taille du récipient réactionnel en verre est de 1 1 à 10 1.

4. Procédé selon la revendication 1, dans lequel sept à dix recyclages de la liqueur mère sont effectués avant de soumettre la liqueur mère usagée aux opérations des étapes (xiii) à (xvi).

5. Procédé selon la revendication 1, dans lequel le diméthoxyéthane est récupéré par distillation et le produit est lavé avec de l'eau dans le cadre de l'opération de l'étape (xv).

6. Procédé selon la revendication 1, dans lequel, dans l'étape (xvi), le rapport molaire entre le $p$-nitrotoluène et le bromure de $p$-nitrobenzyle après le mélange des phases organiques est de 2/1 à 1/3.

7. Procédé selon l'une quelconque des revendications précédentes, dans lequel le rendement en bromure de $p$-nitrobenzyle est de 90 % à 98 % par rapport au $p$-nitrotoluène et l'efficacité des atomes de brome est de 88 % à 92 %.

8. Procédé selon l'une quelconque des revendications précédentes, dans lequel la pureté moyenne du bromure de *p*-nitrobenzyle est supérieure à 98 % (% de surface en CG).

**EP 2 365 960 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6740253 B

**Non-patent literature cited in the description**

- **J. F. BREWSTER.** Bromination of p-nitrotoluene. *J. Am. Chem. Soc.,* 1918, vol. 40 (2), 406-407
- **G. H. COLEMAN.** *Organic Syntheses, Coll.,* 1943, vol. 2, 443
- *ORGANIC SYNTHESES COLL.,* 1936, vol. 16, 54
- **G. W. K. CAVILL.** A note on Bromination of p-nitro-toluene. *J. of the Soc. Of Chem. Industry,* 1946, vol. 40, 124

- **S. ADIMURTHY et al.** *Green Chem.,* 2008, vol. 10, 232
- **ADIMURTHY SUBBARAYAPPA et al.** An alternative method for the region-and stereoselective bromination of alkenes, alkynes, toluene, derevatives and ketones using a bromide/bromated couple. *Green chemistry, Royal Society of Chemistry,* 01 January 2008, vol. 10 (2), 232-237
- **BELL, H. M. ; BROWN, H. C.** *J. Am. Chem. Soc.,* 1966, vol. 88, 1473

15